# EUROPÄISCHE PATENTSCHRIFT

(11) **EP 0 809 657 B1**
(45) Veröffentlichungstag und Bekanntmachung des Hinweises auf die Patenterteilung: **24.10.2001**
(21) Anmeldenummer: 96904067.4
(22) Anmeldetag: 12.02.1996
(51) Int. Cl.: C08B 37/08, C12P 19/26

(54) **VERFAHREN ZUR HERSTELLUNG VON CHITOSAN-GLUKAN-KOMPLEXEN, DARAUS HERSTELLBAREN MASSEN UND DEREN VERWENDUNG**
PROCESS FOR PRODUCING CHITOSAN-GLUCAN COMPLEXES, COMPOUNDS PRODUCIBLE THEREFROM AND THEIR USE
PROCEDE DE FABRICATION DE COMPLEXES CHITOSANE-GLUCANE, PRODUITS OBTENUS A PARTIR DE CES COMPLEXES ET LEUR UTILISATION

(30) Priorität: 13.02.1995 DE 19504640
(43) Veröffentlichungstag der Anmeldung: 03.12.1997
(73) Patentinhaber: Teslenko, Alexander, Dr., 58097 Hagen (DE)
(72) Erfinder: TESLENKO, Alexander, D-58097 Hagen (DE); NIKOLAEWNA, Woewodina, Irina, Abion Beteilungs-und, 52428 Jülich (DE)
(74) Vertreter: Meyers, Hans-Wilhelm, Dr.Dipl.-Chem.
(86) Internationale Anmeldenummer: EP9600590
(87) Internationale Veröffentlichungsnummer: WO9625437

(56) Entgegenhaltungen:
- WO-A-90/14071
- GB-A- 2 026 516
- GB-A- 2 259 709
- DATABASE WPI Week 9622 Derwent Publications Ltd., London, GB; AN 220373 XP002005551 & RU,A,2 043 995 (FOOD AROMAS ACIDS DYES RES INST) , 20.September 1995

## Beschreibung

Gegenstand der vorliegenden Erfindung ist ein Verfahren zur Herstellung von Chitosan-Glukan-Komplexen gemäß Oberbegriff des Anspruchs 1, eine Masse bestehend im wesentlichen aus einem Chitosan-Glukan-Komplex erhältlich durch das erfindungsgemäße Verfahren, daraus geformte Massen, ein Wundheilungsmittel sowie Verwendungen der erfindungsgemäßen Masse.

Zellwände von Mikroorganismen, die Chitin enthalten, werden bereits für viele Anwendungen im Stand der Technik eingesetzt. So ist es möglich mit Mikroorganismen Metallionen aus Wasser zu binden und zu akkumulieren. Die sorptive Potenz der Mikroorganismen wird auf die Zellwände zurückgeführt. Insbesondere Zellwände von mikroskopischen Pilzen, Aktinomyzeten und Hefen weisen verschiedene Komplexe von Polysacchariden auf. So besteht beispielsweise bei Neurospora crassa, Penicillium sp., Aspergillus sp. die innere Schicht der Zellwand aus Chitinmikrofasern, die in einer 1,3-Polyglukanmatrix eingesenkt sind. Die Zellwände bilden eine Schranke gegen viele schädliche Umwelteinflüsse und besitzen eine Schutzfunktion für die entsprechenden Mikroorganismen. So kann der Chitingehalt in Pilzen bis zu 45% der Zellbestandteile (z. B. beim Penicillium digitatum) erreichen. Aus Chitin läßt sich ein wichtiges Aminopolysaccharid, Chitosan, erhalten. Die primären Aminogruppen des Chitosans oder seiner Komplexe können bestimmte Bindungen mit Metallionen selektiv eingehen. Chitosan ist als Sorbens in der Schwermetalleliminierung und als Flockulationsmittel für negativ geladene Kolloide eingesetzt worden. Desweiteren ist es für Polymeren auf Chitinbasis bekannt, daß diese eine Erniedrigung des Cholesteringehaltes bewirken können.

Chitosan könnte eine große Bedeutung für die Anwendung auf vielen Gebieten der Technik erreichen. Bislang ist jedoch kein zufriedenstellendes Verfahren bereitgestellt worden, Chitosanderivate zu erhalten, die beispielsweise in der Medizin, Nahrungsmittelindustrie oder Umwelt- und Entsorgungstechnik eingesetzt werden können. So wird bislang, ausgehend von Myzelien, beispielsweise aus Rhizopus arhisus, das entsprechende Myzel mit 4%-iger Natriumdodecylsulfatlösung, einem aggressiven Detergenz, behandelt, mechanisch zerstört, zentrifugiert und mit Wasser gewaschen. Dadurch ist jedoch eine totale Zerstörung der nativen Zellwandstruktur unvermeidlich. Dies wirkt sich negativ auf das Sorptionsvermögen aus. Es ist desweiteren bekannt, beispielsweise Zellen von Saccharomyces cerevisiae mit denaturierenden Agenzien wie 8 M Harnstofflösung, 1 M Kalilauge und 33 mM Essigsäure-Lösung zu bearbeiten. Hier werden Substanzen erhalten, die nur eine niedrige Sorptionskapazität für Schwermetallionen aufweisen. Diese Methoden sind in Biotechnol. & Bioeng., Vol. 24, N2, Seite 385 - 401, 1982, The Mechanism of Uranium Biosorption by Rhizopus arhisus, The Fungi Eds. Ainsworth G. C., Sussman A. V., Academic Press 1965, p. 46 - 76 sowie in der GB-2,188,135 A1 beschrieben. GB-2,102,506 betrifft die Gewinnung chitinhaltiger Stof-fe aus den Schimmelpilzen Mucor mucedo und Risomucor miecheli. Dabei wird mit 8%-iger H₂O₂-Lösung eine Stunde bei 100°C inkubiert und anschließend mit Natriumsilikatlösung behandelt. Es wird eine Masse erhalten, die einen hohen Protein- und Aschegehalt aufweist. Dies kann sich negativ auf die Flexibilität der entsprechenden Massen auswirken. Die Gewinnung chitinhaltiger Stoffe für den Einsatz in der Wundheilung wird in der US 3,632,575 beschrieben. Dabei erfolgt zunächst eine Chloroformextraktion, eine Behandlung mit 1,0 N Natronlauge, 18 Stunden bei Raumtemperatur, gefolgt von einer Zugabe niedrig konzentrierter Salzsäure und einer Dialyse mit destilliertem Wasser. Hierbei ist die Chloroformextraktion ein großtechnisch problematischer Schritt, da die Verwendung großer Chloroformmengen aus ökologischen und arbeitsplatzhygenischen Gründen nicht zu empfehlen sind.

Die DE 29 23 802 A1 und GB 2,026,516 betreffen ein Verfahren, Myzelien des Schimmelpilzes Mucor rouxii mit 40%-iger Natronlaugelösung bei 128°C vier Stunden lang zu behandeln. In ähnlicher Weise werden beispielsweise Myzelien von Apergillus niger mit 40%-iger Natronlauge 6 Stunden lang behandelt. Man erhält einen Chitosan-Glukan-Komplex von 32 bis 52% des Gesamtgewichts der Pilzmyzelien, wobei der erhaltene Chitin-Glukankomplex Schwermetallionen, z. B. Kupfer, binden kann. Nachteilig an diesem Verfahren ist jedoch, daß es unmöglich ist, den geforderten Reinheitsgrad betreffend Proteine, Fette und Carbonate in diesem Komplex zu gewährleisten. Ein hoher Carbonatgehalt wirkt sich ebenfalls negativ auf die Flexibilität und Anwendbarkeit der erhaltenen Massen aus. Des weiteren ist nachteilig, daß die native Struktur der Schimmelpilzzellwände vollständig zerstört wird und eine Regulierung des Deacetylierungsgrades von Chitin ebenfalls nicht möglich ist.

Die RU-A-2,043,995 beschreibt ein Verfahren zur Herstellung eines Chitosan-Glukan-Komplexes, wobei die chitinhaltigen Mikroorganismen wie Aspergillus niger, Blakeslia trispora, Aktinomyceten roseum oder P. chrysogenum 2 bis 4 mal bei 60 bis 90°C mit einer Natronlaugen-Lösung behandelt werden. Der Chitosan-Glukan-Komplex kann in der Medizin, Landwirtschaft und Nahrungsmittelindustrie als Sorptionsmittel für Metall-Ionen in der Wasserreinigung verwendet werden.

Das der Erfindung zugrundeliegende technische Problem besteht darin, ein Verfahren anzugeben, das zunächst in allgemeiner Form die Nachteile des Standes der Technik vermeidet, also insbesondere in der Lage ist, die Pilzmyzelien so aufzuarbeiten, daß eine möglichst weitgehende Verwendung in unterschiedlichen Bereichen der Technik möglich wird sowie insbesondere den Deacetylierungsgrad zu steuern. Desweiteren soll das Verfahren umweltmäßig unbedenklich und dabei wirtschaftlich sein.

Überraschenderweise wird das der Erfindung zugrundeliegende Problem gelöst durch ein Verfahren gemäß den Merkmalen des Anspruchs 1. Die Unteransprüche 2 bis 6 betreffen bevorzugte Ausführungsformen des erfindungsgemäßen Verfahrens.

Beim erfindungsgemäßen Verfahren zur Herstellung von Chitosan-Glukan-Komplexen geht man von chitinhaltigen Mikroorganismen aus. Diese werden mit Lösungen behandelt, wobei das erfindungsgemäße Verfahren dadurch gekennzeichnet ist, daß die chitinhaltigen Mikroorganismen mindestens zweimal mit einer Laugen-Lösung geringer Konzentration behandelt werden. Danach wird das aus diesem Schritt erhaltene Produkt mit niedrigkonzentrierter Salzsäure, Salpetersäure, Ortho-Phosphorsäure behandelt. An diesen Schritt schließt sich eine Weiterbehandlung mit Natron- oder Kalilaugen-Lösung an, deren Alkalität größer als diejenige der ersten alkalischen Lösung ist. Die Alkalität der zweiten alkalischen Lösung ist so eingestellt, daß eine Deacetylierung des aus dem vorhergehenden Schrittes erhaltenen Produktes nicht vollständig erfolgt. Danach schließt sich das Abtrennen der festen Bestandteile aus dieser Behandlungsstufe an gefolgt vom Verwerfen des Überstandes, Neutralisation des Rückstandes und Waschen des Rückstandes mit Wasser.

Das erfindungsgemäße Verfahren besitzt den Vorteil, das es in schonender Weise die Pilzmyzelien modifiziert, so daß zum einen die native Struktur der Zellwände im wesentlichen erhalten bleibt und der Deacetylierungsgrad steuerbar ist. Dies ist insbesondere von Vorteil zur Einstellung der Eigenschaften der mit dem erfindungsgemäßen Verfahren erhältlichen erfindungsgemäßen Massen, um diese verschiedensten Anwendungsbereichen zuzuführen. So ist zum Beispiel ein höherer Acetylierungsgehalt wünschenswert, wenn die erfindungsgemäßen Massen, beispielsweise zur Wundheilung eingesetzt werden. Soll jedoch das aus dem erfindungsgemäßen Verfahren erhältliche Produkt beispielsweise als Sorbens eingesetzt werden, kann es vorteilhaft sein, den Deacetylierungsgrad zu erhöhen, um über freie Aminogruppen weitere Komplexierungsstellen bzw. Sorptionsstellen zu erhalten.

Im erfindungsgemäßen Verfahren werden vorzugsweise chitinhaltige Mikroorganismen, wie Zygomyzeten, Ascomyzeten, Plectomyzeten, Streptomyzeten, Pyrenomyzeten, Discomyzeten und/oder Hefen eingesetzt. Dazu gehören insbesondere Aspergillus sp., Penicillium sp., Mucor sp., Phycomyces sp., Choanephora sp., Zygorrhynchus sp., Blakesila sp., Actinomyces sowie Saccharomyces sp. Vorzugsweise werden die genannten Mikroorganismen im Stadium ihres höchsten Chitingehaltes und/oder im Endzustand ihrer Anwendung für industrielle Zwecke eingesetzt. Grundsätzlich kommt es jedoch auf den Entwicklungszustand nicht an. Diese Pilze bzw. Hefen zeichnen sich durch einen Chitingehalt in ihrer Zellwand aus.

Erfindungsgemäß wird bevorzugt, daß die Behandlung mit einer 2 bis 4% oder 8%-igen Natronlaugen-Lösung erfolgt.

Die Temperaturen liegen vorzugsweise im Bereich von 50 bis 110°C und insbesondere bei 60 bis 90°C.

Vorzugsweise beträgt die Normalität der zu verwendenden Mineralsäure im Schritt b) des Anspruchs 1 0,5 bis 5. Als Mineralsäure kommen Salzsäure, Salpetersäure, ortho-Phosphorsäure oder Kombinationen davon in Frage. Weniger bevorzugt ist Schwefelsäure, da bei Behandlung mit Schwefelsäure eine Tendenz zur Ausfällung von Sulfaten nicht immer zu verhindern ist.

Vorzugsweise beträgt die Konzentration der Natron- oder Kalilaugen-Lösung gemäß Schritt c) des Anspruchs 1 20 bis 30%. Die Behandlungstemperatur liegt zwischen 110 und 125°C und die Behandlung dauert vorzugsweise zwischen 30 und 180 min., insbesondere zwischen 60 und 120 min., und besonders bevorzugt zwischen 60 und 90 min. Erfindungsgemäß werden die chitinhaltigen Myzelien der Schimmelpilze also vor ihrer Deacetylierung mit weniger konzentrierter Laugenlösung behandelt, um beispielsweise Pigmente, Fette und Proteine zu eliminieren. Die Demineralisierung erfolgt mit niedrig konzentrierter Mineralsäure, vorzugsweise bei Raumtemperatur. Die so bearbeiteten Pilzmyzelien werden dann mit der zweiten alkalischen Lösung deacetyliert. Aus elektronenmikroskopischen Untersuchungen ergibt sich, daß nach dem erfindungsgemäßen Verfahren die native Struktur der Zellwände weitestgehend erhalten bleibt. Die erfindungsgemäß geforderte Demineralisation gemäß Schritt b) des Anspruchs 1 entfernt Salze von den Zellwänden, vorzugsweise nach einer Behandlungszeit von 2 Stunden bei Raumtemperatur.

Die Vorteile des erfindungsgemäßen Verfahrens liegen darin, daß die damit gewinnbaren Chitosan-Glukan-Komplexe nur einer relativ geringen Laugenkonzentration ausgesetzt waren, daß kurze Inkubationszeiten eingehalten werden können, daß Verbesserungen des Reinheitsgrades des Komplexes erzielbar sind, die nativen Strukturen der Zellwand erhalten bleiben. Dadurch lassen sich hocheffektive neue Sorbentien zur Verfügung stellen, die biologisch abbaubar und nicht toxisch sind. Damit eröffnen sich neue Einsatzgebiete dieser Materialien, zum Beispiel in der Medizin, Nahrungsmittelindustrie und dem Umweltschutz.

Gegenstand der vorliegenden Erfindung sind mithin durch das erfindungsgemäße Verfahren erhältliche Massen, die im wesentlichen aus einem Chitosan-Glukan-Komplex bestehen. Im wesentlichen bedeutet hier, daß auch noch geringe Mengen anderer Substanzen, insbesondere Chitin oder mit Acetylgruppen oder Restmineralien, in dem Material enthalten sein können. Der Reinheitsgrad ist jedoch sehr hoch.

Die nach dem erfindungsgemäßen Verfahren erhältlichen erfindungsgemäßen Massen zeichnen sich insbesondere dadurch aus, daß die Struktur der Zellwand der Mikroorganismen, aus der die Massen stammen, noch weitestgehend intakt sind. Der Ascheanteil dieser Materialien liegt unter 10%. Dies ist ebenfalls eine Angabe, die den Begriff "im wesentlichen" für den Fachmann hinreichend erläutert.

Die erfindungsgemäßen Massen lassen sich in ihrem Deacetylierungsgrad mittels des erfindungsgemäßen Verfahrens beeinflussen. So kann durch eine Erhöhung der Expositionszeit des Myzels mit Erhöhung der zweiten alkalischen Lösung eine erhöhte Deacetylierung erzielt werden, wohingegen die Behandlung mit geringerer Laugenkonzentration oder bei niedrigerer Temperatur innerhalb kürzerer Zeit ein geringerer Deacetylierungsgrad einstellbar wird.

Aus den erfindungsgemäßen Massen lassen sich geformte Massen herstellen, indem beispielsweise eine Suspension der erfindungsgemäßen Masse einer Gefriertrocknung unterzogen wird. Die geformten Massen können insbesondere in Form von porösen Filmen, wie Membranen, Matten, Pflastern, Vliesen, insbesondere in Form sogenannter Non-Woven-Textilien, oder Fäden vorliegen. Die geformten Massen können auch aus einer ersten Masse, die aus der erfindungsgemäßen Masse besteht, und einer zweiten Zellulose enthaltenden Masse geformt sein. Insbesondere können solche geformten Massen in Form einer Folie oder einer blattartigen Struktur vorliegen. Aus den geformten oder den ungeformten Massen gemäß der Erfindung lassen sich insbesondere Wundheilungsmittel herstellen, vorzugsweise in Folien- oder Streifenform. Diese können dann als Pflaster auf eine Wunde aufgelegt, die Heilung dieser Wunde beschleunigen. Wird die erfindungsgemäße Masse in Fäden geformt, können diese als chirurgische Wundnahtmaterialien eingesetzt werden.

Als weitere Anwendungsgebiete kommen die erfindungsgemäßen Massen als Enterosorbentien in Frage. Darunter sind Sorbentien zu verstehen, die aus Körperflüssigkeiten, wie Blutserum, oder im Magen Substanzen zu binden vermögen. Dazu gehören beispielsweise Cholesterin oder andere Stoffe, die z. B. in pathologischen Fällen in erhöhter Konzentration auftreten. Ebenso lassen sich andere Körperflüssigkeiten, wie beispielsweise Urin mit diesen Sorbentien reinigen. Es können aufgrund der sorptiven Potenz insbesondere Bakterien und Toxine, unerwünschte Enzyme etc. gebunden werden. Auf der sorptiven Potenz der erfindungsgemäßen Massen beruht auch der Anwendungsbereich als Wundabdeckung, wodurch beispielsweise eine Gewebeentwässerung erfolgt. Vorteilhaft wirkt sich hierbei insbesondere die Einwirkung hydrolysierender Enzyme, wie Lysozym, auf die erfindungsgemäßen Massen aus. Dabei entstehen Oligosaccharide, die eine Heilwirkung aufweisen können, wie beispielsweise Oligomere aus oder mit N-Acetyl-Glukanamin. In solchen Fällen kann es also sinnvoll sein, den Deacetylierungsgrad möglichst gering zu halten, um eine hohe Freisetzung der gewünschten Oligosaccharide zu gewährleisten. Aufgrund des sorptiven Charakters kann die erfindungsgemäße Masse bateriostatisch oder gar bakterizid wirken, indem Bakterien adsorbiert werden. Dies ist insbesondere vorteilhaft bei der Verwendung der erfindungsgemäßen Massen als Wundabdeckung oder Enterosorbens. Die letztgenannte Eigenschaft qualifiziert die erfindungsgemäßen Massen jedoch auch als Stabilisator für Nahrungsmittel, insbesondere Emulsionen.

Die erfindungsgemäßen Massen sind in geformter Form, beispielsweise als Membranen oder Presskörper, wie auch in Schüttungen zur Entfernung und/oder Rückgewinnung von Substanzen, insbesondere aus Lösungen geeignet. Als zu sorbierende Substanzen kommen sowohl organische wie auch anorganische Substanzen in Frage. So können beispielsweise Schwermetalle aus wäßrigen Lösungen entfernt werden, so daß die erfindungsgemäßen Massen im Umweltschutz eingesetzt werden können. Es lassen sich ebenfalls organische Substanzen, insbesondere Pestizide, Herbizide und andere organische Schadstoffe aus Lösungen entfernen. Auch in diesem Anwendungsbereich können die erfindungsgemäßen Massen im Umweltschutz zur Geltung kommen.

Eine weitere interessante Anwendung besteht in der Verwendung als Chromatographiehilfe zur Trennung oder Immobilisierung von Biopolymeren, wie Enzymen, Antikörpern und Katalysatoren. So zeigen Adsorptionsdaten von Übergangsmetallionen, daß diese auch mit dem Komplex von Alkali- und Erdalkalielementen getrennt werden können. Wird Lysozym beispielsweise an dem Chitosan-Glukan-Komplex chromatographisch in einer Säule getrennt, so behält es 70% seiner Aktivität.

Desweiteren kommen noch andere Anwendungen der erfindungsgemäßen Massen in Frage, so beispielsweise zur Herstellung von Produkten und Zubereitungen, wie Leimen, Klebemitteln, Flockungsmitteln, Koagulationshilfen, Substraten für Enzyme, Adsorptionsmittel für chromatographische Zwecke, Fertigungsmittel, Zusätze zu Papier, Hilfsstoffe und Zusätze für Textilien, für Glaswolle, für Kunststoffe, für Farbstoffe für natürliche und künstliche Häute, für pharmazeutische Kapseln, zur Zellaggregation, für mikrobiologische Medien, für Kulturmedien sowie Nahrungsmittel für Menschen und Tiere in Frage.

Durch den guten Reinheitsgrad der hergestellten Chitin-Chitosan-Glukan-Komplexe, d. h. den niedrigen Gehalt an Protein und Melanin und den niedrigen Gehalt an Mineralien, vor allem Carbonaten (Ascheanteil) und den hohen und/oder einstellbaren Deacetylierungsgrad und durch die weitgehende Erhaltung der Zellwandstruktur lassen sich definierte Produkte mit definierten Eigenschaften erhalten. Die herausragende Eigenschaft ist insbesondere die hohe Sorptionskapazität (siehe Tabelle 4), die durch alle drei Behandlungsschritte positiv beeinflußt wird, für viele Stoffe aus Lösungen, wie biologischen Flüssigkeiten (Blut, Serum), Abwässern usw. zur Entfernung, Lagerung und/oder Wiederverwertung. Außerdem ist das Produkt nicht toxisch und biologisch abbaubar.

Zu den in der Praxis relevanten Verwendungen gehört die Sorptionsfähigkeit insbesondere für Schwermetalle, inklusive Uran und anderen Radionukliden, sowie die für Bakterien und deren Toxine und Metaboliten, Proteine und Fette und andere organische und biologische Verbindungen, wie Cholesterol, Harnstoff, Pestizide, Dioxin, Farbstoffe, Aldehyde, Säuren usw.

Für verschiedene Verwendungen kann der Chitin-Chitosan-Glukan-Komplex mit erhaltener Zellwandstruktur nicht nur als Pulver oder Granulat, sondern auch leicht in anderen Formen wie Film, Folie, Membran (selbsttragend), Papier, "Löschpapier" (insbesondere im Komposit mit Cellulose), schwammartige oder gewebeartige Struktur (durch Gefriertrocknung, z. B. Pflaster) hergestellt werden. Weitere Anwendungen sind als Flockungsmittel für Polyelektrolyte und Kolloide sowie als Stabilisatoren für Emulsionen.

In der Medizin ergeben sich mehrere Anwendungsmöglichkeiten des Produktes aufgrund seiner Eigenschaften. Aus der hohen Sorptionskapazität für unerwünschte Stoffe und der Ungiftigkeit, die sich im Tierversuch erwiesen hat, ergibt sich die Anwendung als Enterosorbens für die Reinigung des Magen-Darm-Traktes oder des Bluts sowie die Senkung der Konzentration bestimmter Stoffe im Blut, wie Cholesterol.

Die gewebeartige Struktur erlaubt die Herstellung von elastischen hochporösen, d. h. gut luftdurchlässigen Pflastern für die Wundabdeckung. Es lassen sich sichtbare Poren erzeugen. Die Porosität läßt sich durch Begasung während der Gefriertrocknung einstellen. Durch Zusatz gewünschter Stoffe kann das Pflaster als Depot für diese dienen. Der Abbau von Chitin-Chitosan-Strukturen durch Hydrolyse (z. B. mit Lysozym) setzt direkt auf dem Pflaster Oligosaccharide enthaltend N-Glukosamin- und N-Acetylglucosamineinheiten frei, die an der Geweberegeneration teilnehmen und die Wundheilung beschleunigen. Die Zellproliferation wird aktiviert, die Epithelbildung stimuliert und die Bildung von Kollagenphasen verringert (Heilung großer Wunden ohne Narben). Durch die Sorption von Bakterien wirkt das Pflaster "bakterizid". Positiv wirkt auch die Sorption von Entzündungsmediatoren und proteolytischen Enzymen.

Die hohe Wasseraufnahmekapazität (bei der Trocknung verliert das Produkt im Verhältnis zum Trockengewicht 500% an Wasser) kann das Material zur Gewebeentwässerung und Wunddrainage eingesetzt werden.

Die Erfindung wird anhand der folgenden Beispiele näher erläutert.

### Beispiel 1

Herstellung eines Chitosan-Glukan-Komplexes aus A.niger aus Abfall der Zitronensäureproduktion.
1. Entfernung von Protein u.ä.: Zu 150 g Myzelien (Feuchtgewicht) von A. niger in einem chemischen Reaktor von 2 l Gesamtvolumen wurden 1,5 l 8%-ige Natronlauge zugefügt. Die erhaltene Dispersion wurde 60 min. bei 90°C gerührt (60 rpm). Danach wurde der Inhalt durch einen Gewebefilter filtriert. Der Filterkuchen wurde noch einmal mit 1,5 l 8%-iger Natronlauge 60 min. bei 90°C gerührt. Das erhaltene Produkt wurde filtriert und dreimal mit Wasser im 20fachen Überschuß gewaschen.
2. Entfernung von Mineralien (vor allem Carbonat): Das Produkt wurde im Reaktor mit 1,5 l 2N Salzsäure 120 - 180 Minuten bei Raumtemperatur behandelt, anschließend filtriert und mit destilliertem Wasser bis zur Neutralität gewaschen.
   Es wurden 90 - 95 g (Feuchtgewicht) Chitin-Glukan-Komplex aus A. niger-Myzelien (oder 18 - 19 g Trockengewicht) erhalten.
3. Deacetylierung: Das Produkt aus 2. wurde in einem Reaktor mit 1,0 l 29%-iger Natronlauge 90 Minuten bei 125°C gerührt. Danach wurde die Masse über Glasgewebe filtriert. Zu dem Filterkuchen wurden in einem Reaktor unter Rühren 4 l destilliertes Wasser zugefügt. Zu der erhaltenen Dispersion wurde im Laufe von 180 - 240 Minuten kontinuierlich 4%-ige Salzsäure gegeben, bis ein pH-Wert von 6,5 - 7,0 erreicht ist. Das neutralisierte Produkt wurde filtriert und mit destilliertem im 10fachen Überschuß gewaschen. Es wurden 65 g (Feuchtgewicht) Chitin-Chitosan-Glukan-Komplex aus A. niger-Myzelien (oder 17 g Trockengewicht) erhalten. Das Produkt war farblos. Insbesondere in der Dispersion war zu sehen, daß die Zellwände erhalten geblieben waren, was durch elektronenmikroskopische Aufnahmen bestätigt wurde. Der Deacetylierungsgrad bezogen auf das Chitin im Chitin-Glukan-Komplex war 89%.

### Beispiele 2 - 11

Herstellung von Chitosan-Glukan-Komplexen aus A. niger aus Abfall der Zitronensäureproduktion.

Grundsätzlich wie Beispiel 1. Tabelle 1 gibt die unterschiedlichen Bedingungen an.

Beispiel 10 wurde nach DE-A-29 23 802 hergestellt ("Prototyp).

Beispiel 11 zeigt eine Herstellung in pilottechnischem Maßstab entsprechend Nr. 1: 6 kg Myzelien mit 60 l 8%-ige Natronlauge im ersten Schritt und 60 l 2 N Salzsäure im zweiten Schritt ergeben 4,9 kg Chitin-Glukan-Komplex, daraus werden mit 50 l 29%-iger Natronlauge 4,5 kg (Feuchtgewicht) Chitin-Glukan-Komplex entsprechend 0,8 kg Trockengewicht.

Entsprechend den Beispielen 1 - 9 wurden auch Chitin-Chitosan-Glukan-Komplexe aus Myzelien von P. chrysogenum (Nr. 12 - 20), Blakeslia trispora (Nr. 21 - 29) und Streptomyces sp. (Nr. 30 - 38) hergestellt.

Tabelle 2 zeigt die Wirkung der Proteinentfernung und der Demineralisierung (Carbonatentfernung). Der Proteingehalt im Chitin-Glukan-Komplex wurde mit dem Folin-Reagenz bei 750 nm (niedriger Konzentrationsbereich) und 500 nm (hoher Konzentrationsbereich) bestimmt. Der Proteingehalt wurde aus einer Eichgeraden mit Rinderserumalbumin-Standards nach R.M.C. Dawson et al., "Data for Biochemical Research", Clarendon Press, Oxford 1986, Seite 550, bestimmt, der Melaningehalt spektrophotometrisch nach A. Malama, "Biologisch aktive Stoffe aus Mikroorganismen", in Nauka i Technika, Minsk, 1975, Seiten 120- 126.

Tabelle 3 zeigt die Wirkung der Deacetylierung. Der Deacetylierungsgrad wurde mit UV-Spektroskopie nach R.A.A. Muzzarelli, G. Rochette, "Determination of the Degree of Deacetylation of Chitin by First Derivative UV-Spectrometry", in Carbohydr. Res. 1985, 5, Seiten 461 - 472 bestimmt. Die angegebene Kationaustauschkapazität bezieht sich auf das Gleichgewicht. Die Nr. 10 ist der "Prototyp" nach DE 29 23 802.

Aus den Daten ist zu erkennen, daß der Deacetylierungsgrad mit der Reaktionsdauer zunimmt, daß aber auch bei zu hohen Reaktionszeiten (in Abhängigkeit von der Laugenkonzentration und Temperatur) die Zerstörung der Zellwände zunimmt. Mit der Reaktionsdauer nimmt auch der Anteil der wasserlöslichen Fraktion des Chitin-Chitosan-Glukan-Komplexes zu. Die chemischen Verschiebungen im ¹H-NMR-Spektrum der löslichen Fraktion und die Titration der Aminogruppen zeigen, daß es sich um Chitosan handelt.

Tabelle 4 zeigt die Gleichgewichts-Sorptionskapazitäten für Schwermetallkationen, die Farbstoffe Aktiv Rot und Aktiv Blau sowie das Protein Rinderserumalbumin. Die Sorptionsexperimente wurden mit 0,1 g Chitosan-Glukan-Komplex in 50 ml durchgeführt, wobei Schwermetallkationen in Konzentrationen von 52 mg/ml eingesetzt wurden (als Salze wurden verwendet: Pb(NO₃)₂ Hg(Ac)₂, K₂Cr₂O₇ und CuSO₄), die Farbstoffmenge betrug etwa 70 mg/ml und die Proteinmenge etwa 10 mg/ml. Die Werte liegen deutlich über denen, die in der DE 29 23 802 für den dort hergestellten Chitosan-Glukan-Komplex (10*, Tabelle 4) genannt wurden, die denen entsprechen, die für den nach den dortigen Angaben hergestellten Prototyp Nr. 10 gefunden wurden.

Außerdem ist für zwei Komplexe die prozentuale Abnahme der Cholesterinkonzentration aus 50 ml Lösungen mit 100 mg/l und 0,1 g Chitosan-Glukan-Komplex angegeben.

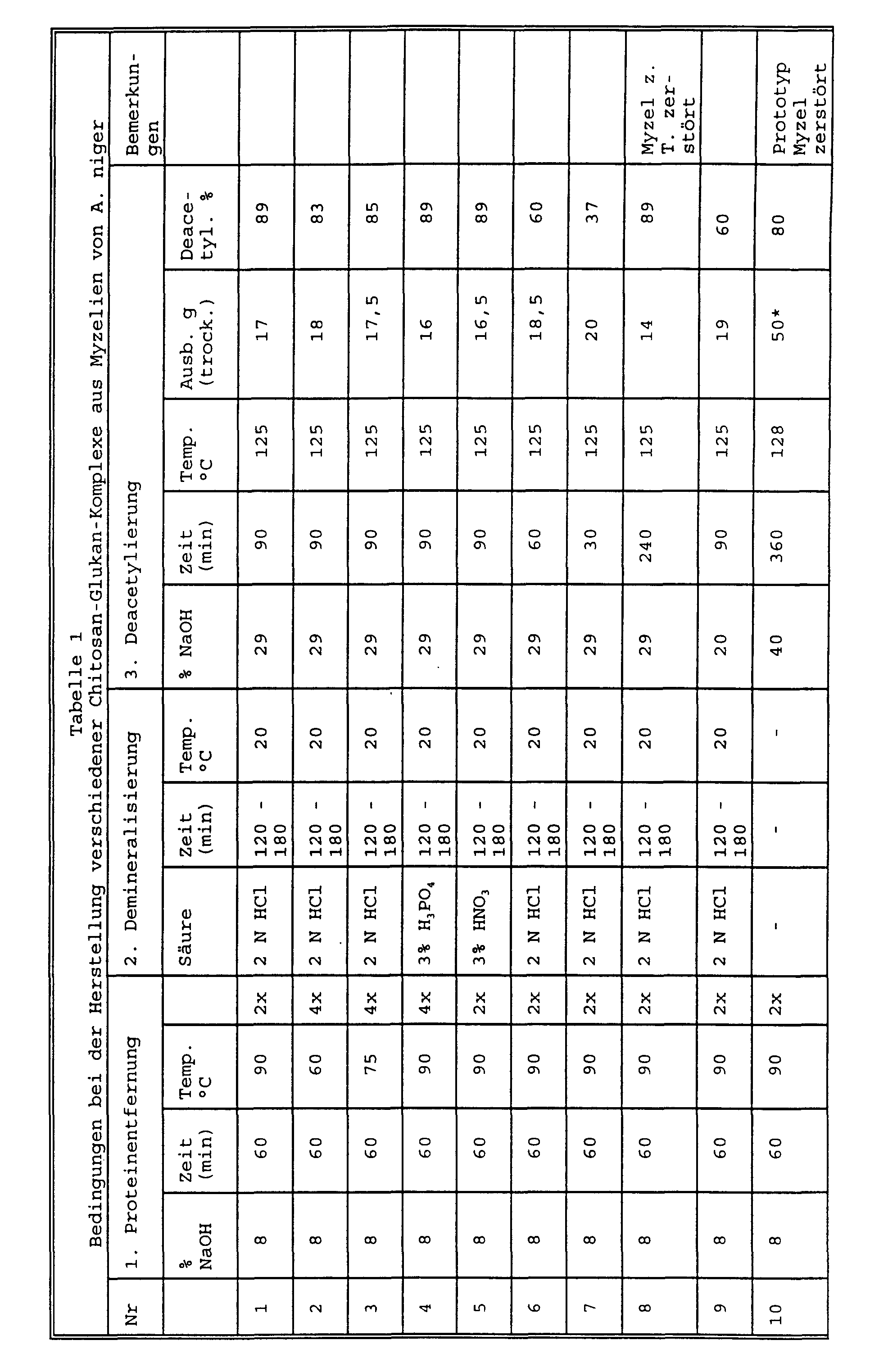

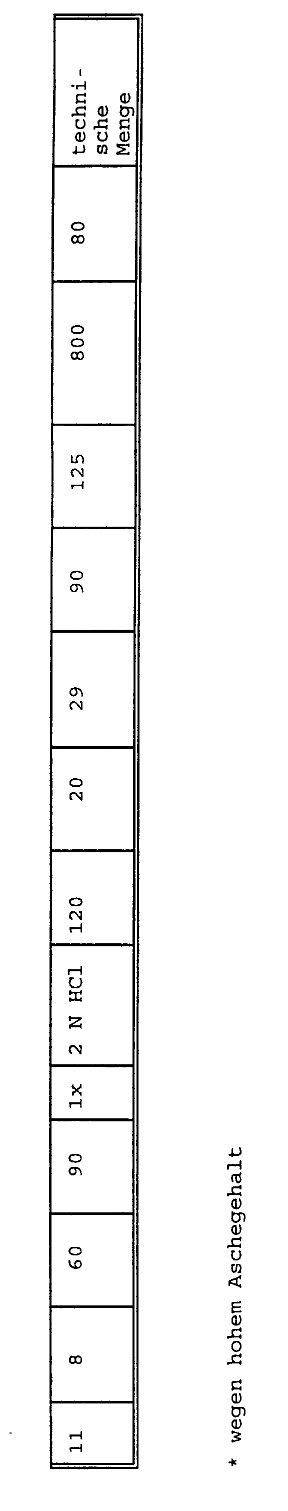

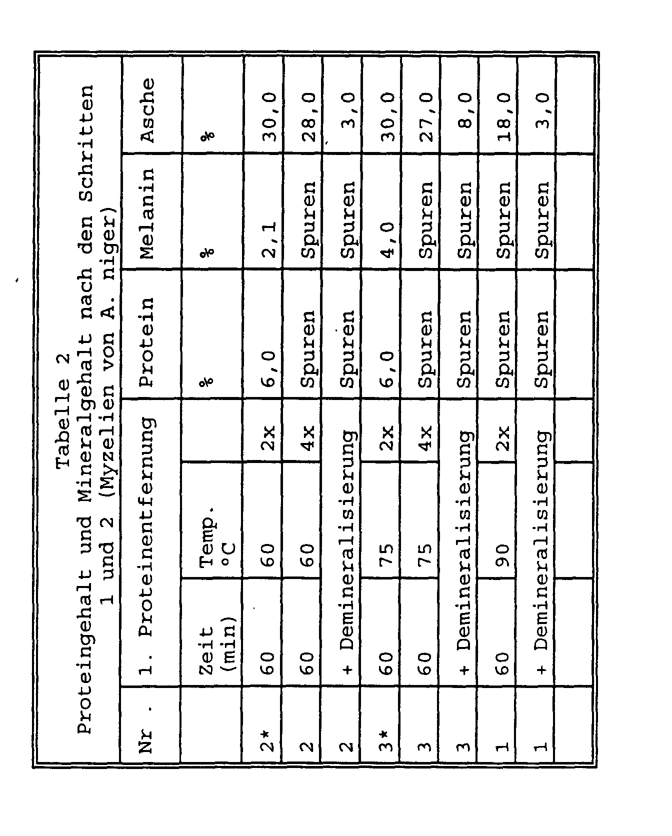

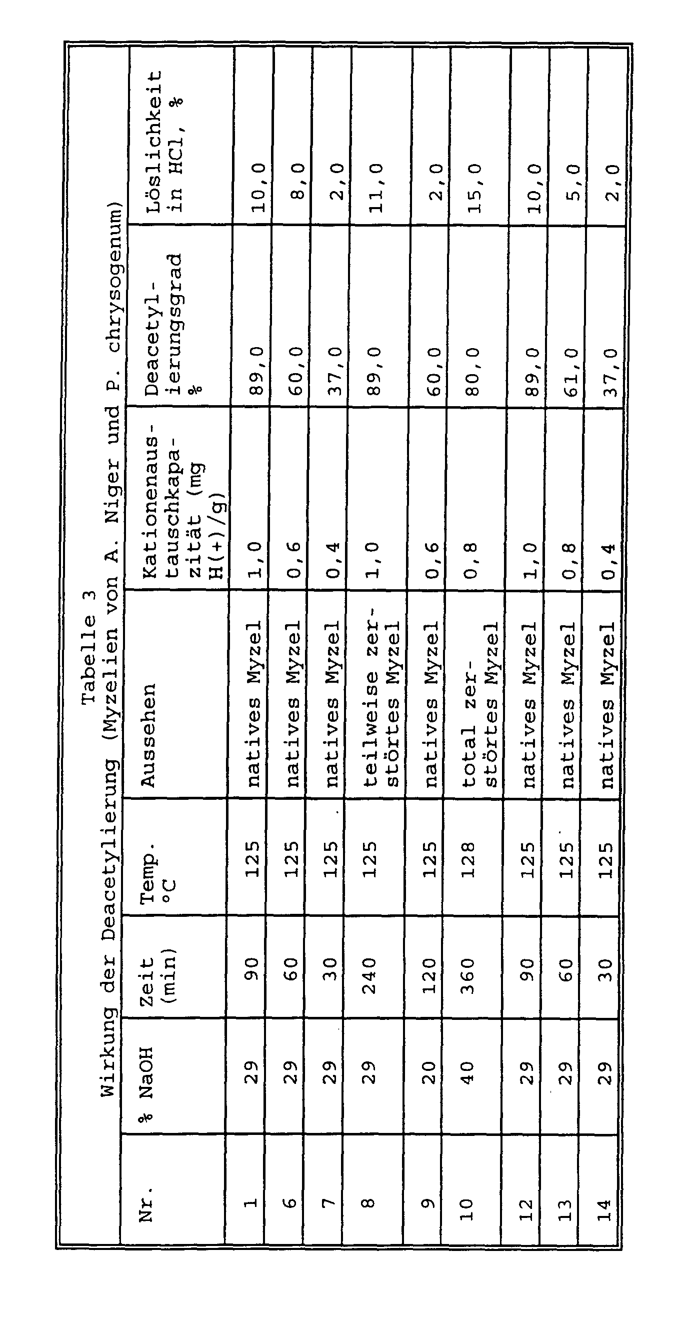

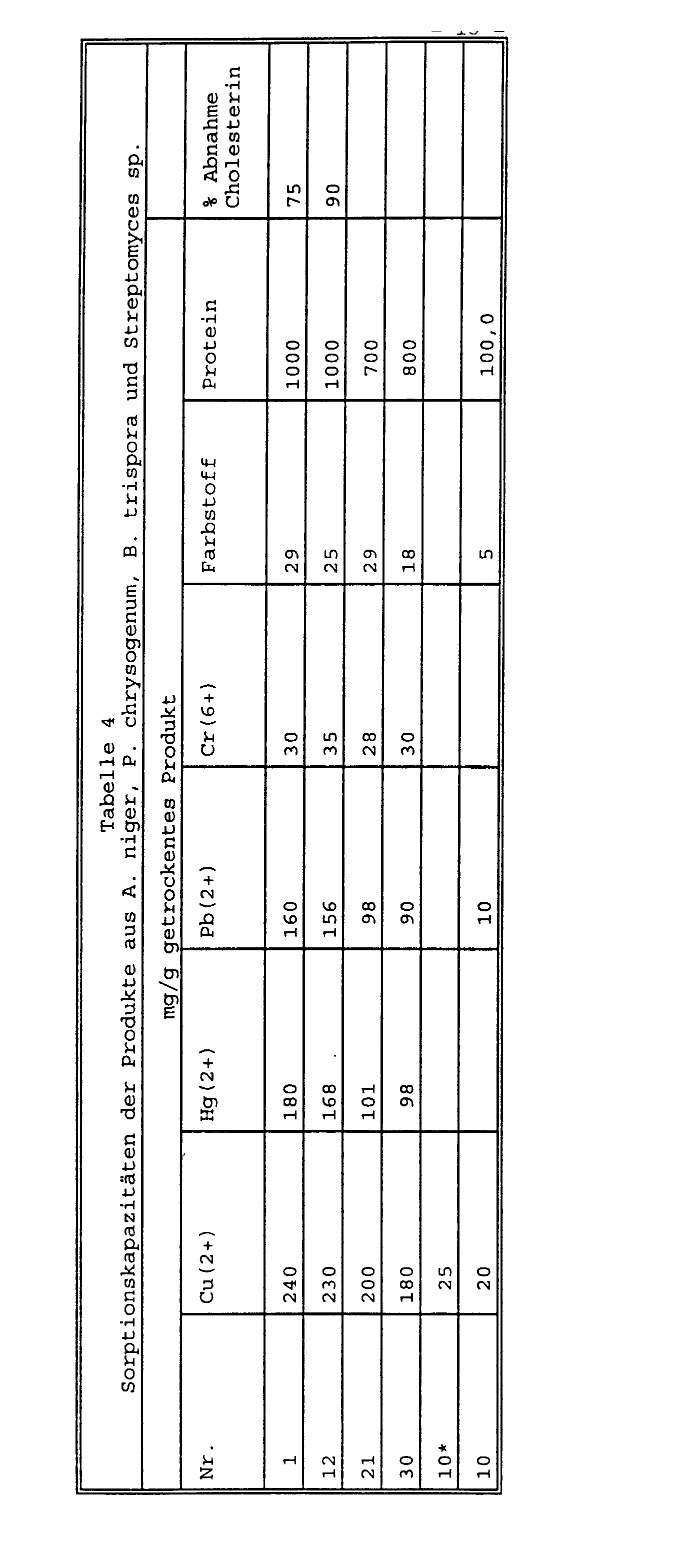

## Patentansprüche

1. Verfahren zur Herstellung von Chitosan-Glukan-Komplexen aus chitinhaltigen Mycelien von mikroskopischen Pilzen durch Behandlung der Mycelien mit Lösungen, **dadurch gekennzeichnet, dass**
a)chitinhaltige Mikroorganismen mindestens zweimal mit einer Laugen-Lösung geringer Konzentration behandelt werden,
b)danach das aus Schritt a) erhaltene Produkt mit niedrig konzentrierter Salzsäure, Salpetersäure, ortho-Phosphorsäure behandelt wird,
c)woraufhin eine weitere Behandlung mit Natron- oder Kalilaugen-Lösung für 60 bis 120 min erfolgt, deren Alkalität größer als diejenige der alkalischen Lösung aus Schritt a) ist, zur nicht vollständigen Deacetylierung der aus Schritt b) erhaltenen Fraktion,
d)Abtrennen der festen Bestandteile, Verwerfen des Überstandes, Neutralisation und Waschen des Rückstandes mit Wasser.

2. Verfahren nach Anspruch 1, **dadurch gekennzeichnet, dass** die chitinhaltigen Mikroorganismen Aktinomyceten, Aspergillus, Penicillium, Blakeslia, und/oder Hefen sind.

3. Verfahren nach Anspruch 1 und/oder 2, **dadurch gekennzeichnet, dass** die Behandlung mit einer 8%-igen Natronlaugen-Lösung durchgeführt wird.

4. Verfahren nach mindestens einem der Ansprüche 1 bis 3, **dadurch gekennzeichnet, dass** die Normalität der Mineralsäure 2N ist.

5. Verfahren nach mindestens einem der Ansprüche 1 bis 4, **dadurch gekennzeichnet, dass** die Konzentration der Natron- oder Kalilaugen-Lösung 29 % beträgt.

6. Verfahren nach mindestens einem der Ansprüche 1 bis 5, **dadurch gekennzeichnet, dass** im Schritt c) die Behandlung zwischen 30 oder 60 min dauert.

7. Masse bestehend im wesentlichen aus einem Chitosan-Glukan-Komplex erhältlich durch ein Verfahren nach mindestens einem der Ansprüche 1 bis 6.

8. Masse nach Anspruch 7, **dadurch gekennzeichnet, dass** die Masse die Struktur der Zellwand der Mikroorganismen, aus der die Masse stammt, aufweist.

9. Masse nach Anspruch 7 und/oder 8, **dadurch gekennzeichnet, dass** die Masse einen Ascheanteil von 3% oder 8% aufweist.

10. Verwendung einer Masse nach mindestens einem der Ansprüche 7 bis 9 als Sorptionsmaterial zur Entfernung und/oder Rückgewinnung von Substanzen, insbesondere von Schwermetallen aus Lösungen.

11. Verwendung einer Masse nach mindestens einem der Ansprüche 78 bis 9 als biologisch abbaubares, nicht toxisches Sorptionsmittel für Schwermetallionen, Farbstoffen, Proteine und Fette.

12. Verwendung nach Anspruch 11 als Enterosorbens in vivo.

13. Verwendung einer Masse nach mindestens einem der Ansprüche 7 bis 9 als Wundheilungsmittel.

14. Verwendung einer Masse nach mindestens einem der Ansprüche 7 bis 9 als Träger für Wirkstoffe zur allmählichen Abgabe der Wirkstoffe.

## Claims

1. A process for the preparation of chitosan-glucan complexes from chitin-containing mycelia of microscopic fungi by treating the mycelia with solutions, **characterized in that**
a) chitin-containing microorganisms are treated at least twice with a low concentration alkaline solution;
b) followed by treating the product obtained from step a) with low concentration hydrochloric acid, nitric acid, ortho-phosphoric acid;
c) followed by a further treatment for 60 to 120 min with an aqueous sodium or potassium hydroxide solution whose alkalinity is higher than that of said alkaline solution of step a) for incomplete deacetylation of the fraction obtained from step b);
d) separating the solid components, discarding the supernatant, neutralization and washing the residue with water.

2. The process according to claim 1, **characterized in that** said chitin-containing microorganisms are actinomycetes, *Aspergillus, Penicillium, Blakeslea* and/or yeasts.

3. The process according to claim 1 and/or 2, **characterized in that** the treatment is performed with an 8% aqueous sodium hydroxide solution.

4. The process according to at least one of claims 1 to 3, **characterized in that** the normality of the mineral acid is 2 N.

5. The process according to at least one of claims 1 to 4, **characterized in that** the concentration of said aqueous sodium or potassium hydroxide solution is 29%.

6. The process according to at least one of claims 1 to 5, **characterized in that** the treatment in step c) lasts between 30 or 60 min.

7. A mass essentially consisting of a chitosan-glucan complex obtainable by a process according to at least one of claims 1 to 6.

8. The mass according to claim 7, **characterized by** having the structure of the cell wall of the microorganisms from which the mass is derived.

9. The mass according to claim 7 and/or 8, **characterized by** having an ash content of 3% or 8%.

10. Use of a mass according to at least one of claims 7 to 9 as a sorption material for the removal and/or recovery of substances, especially heavy metals, from solutions.

11. Use of a mass according to at least one of claims 7 to 9 as a biodegradable non-toxic sorption agent for heavy metal ions, dyes, proteins and fats.

12. The use according to claim 11 as an enterosorbent in vivo.

13. Use of a mass according to at least one of claims 7 to 9 as a wound-healing agent.

14. Use of a mass according to at least one of claims 7 to 9 as a support for active substances for a sustained release of the active substances.

## Revendications

1. Procédé de préparation de complexes chitosane-glucane à partir de mycéliums contenant de la chitine de champignons microscopiques, par traitement des mycéliums par des solutions, **caractérisé en ce que**
a) on traite au moins deux fois des microorganismes contenant de la chitine avec une solution alcaline à faible concentration,
b) puis on traite le produit obtenu dans l'étape a) avec de l'acide chlorhydrique, de l'acide nitrique, de l'acide orthophosphorique à faible concentration,
c) ce après quoi on procède à un autre traitement avec une solution d'hydroxyde de sodium ou de potasse pendant 60 à 120 min, dont l'alcalinité est supérieure à celle de la solution alcaline de l'étape a), pour une désacétylation incomplète de la fraction obtenue dans l'étape b),
d) on sépare les constituants solides, on rejette le surnageant, on neutralise et on lave le résidu avec de l'eau.

2. Procédé selon la revendication 1, **caractérisé en ce que** les microorganismes contenant de la chitine sont les actinomycètes, Aspergillus, Penicillium, Blakesilla, et/ou des levures.

3. Procédé selon la revendication 1 et/ou 2, **caractérisé en ce que** le traitement est effectué avec une solution de soude à 8 %.

4. Procédé selon au moins l'une des revendications 1 à 3, **caractérisé en ce que** la normalité de l'acide minéral est de 2 N.

5. Procédé selon au moins l'une des revendications 1 à 4, **caractérisé en ce que** la solution d'hydroxyde de sodium ou de potasse est de 29 %.

6. Procédé selon au moins l'une des revendications 1 à 5, **caractérisé en ce que**, dans l'étape c), le traitement dure de 30 à 60 min.

7. Masse constituée pour l'essentiel d'un complexe chitosane-glucane, pouvant être obtenu par un procédé selon au moins l'une des revendications 1 à 6.

8. Masse selon la revendication 7, **caractérisée en ce que** la masse présente la structure de la paroi cellulaire des microorganismes dont provient la masse.

9. Masse selon la revendication 7 et/ou 8, **caractérisée en ce que** la masse contient de 3 à 8 % de cendres.

10. Utilisation d'une masse selon au moins l'une des revendications 7 à 9 en tant que sorbant pour éliminer et/ou récupérer des substances, notamment des métaux lourds, à partir de solutions.

11. Utilisation d'une masse selon au moins l'une des revendications 7 à 9, en tant que sorbant non toxique biodégradable pour ions de métaux lourds, colorants, protéines et graisses.

12. Utilisation selon la revendication 11 en tant qu'entérosorbant in vivo.

13. Utilisation d'une masse selon au moins l'une des revendications 7 à 9 en tant qu'agent de cicatrisation.

14. Utilisation d'une masse selon au moins l'une des revendications 7 à 9 en tant que support pour principes actifs, pour la libération progressive des principes actifs.
